# EUROPEAN PATENT SPECIFICATION

(11) **EP 2 802 863 B1**
(45) Date of publication and mention of the grant of the patent: **03.01.2024**
(21) Application number: 13703519.2
(22) Date of filing: 09.01.2013
(51) Int. Cl.: G01N 21/93

(54) **AUTOMATIC STANDARDS INSPECTION**
AUTOMATISCHE INSPEKTION VON BEZUGSOBJEKTEN
INSPECTION AUTOMATIQUE D'OBJETS STANDARD

(30) Priority: 09.01.2012 DK 201270012
(43) Date of publication of application: 19.11.2014
(73) Proprietor: Stevanato Group Denmark A/S, 8600 Silkeborg (DK)
(72) Inventor: NIELSEN, Gert, 8240 Risskov (DK)
(74) Representative: Patentgruppen A/S
(86) International application number: PCT/DK2013/050006
(87) International publication number: WO 2013/104365

(56) References cited:
- US-A- 4 154 672
- US-A1- 2010 282 650
- Anonymous: "Gauge (instrument) - Wikipedia", , 14 October 2019 (2019-10-14), XP055655860, Retrieved from the Internet: URL:https://en.wikipedia.org/wiki/Gauge_(i nstrument) [retrieved on 2020-01-08]

## Description

### Field of the invention

The present invention relates to inspection machines for quality control of containers such as ampoules, vials, cartridges and syringes for pharmaceutical products and their contents, e.g. solutions or suspensions, and in particular to the verification of such inspection machines.

### Background of the invention

Several methods and apparatuses for inspecting containers and the contents thereof are known in the art. Particularly in the field of manufacture and distribution of medicaments the tolerances regarding the number of contaminated entities or imperfect containers are zero or at least very low. Hence the requirements regarding, e.g., inspection systems for such manufacture processes are high, and there is often a noticeable dependency between the quality of the inspection system, especially its false rejection rate, i.e. the number of flawless entities falsely rejected, and the profit obtainable from the process.

Modern inspection machines use advanced vision technology and image processing as well as other inspection technologies such as high voltage leak detection to automatically detect several kinds of defects. An inspection machine typically comprises a number of inspection stations that utilize the above-mentioned technologies in different ways to determine up to currently about 25 different parameters each representing a different kind or aspect of a defect, e.g. cap closure, cracks in the container or foreign particles in the content. The containers with their contents are conveyed through the different inspection stations until they are eventually divided into good products and rejected products on the basis of the parameter values determined for each container. The different inspection stations typically employ complex setups of high-precision optics, innovative illumination, accurate and fast movable mirrors, positioning systems and spin motors, state-of-the-art line or array scanners, etc., all computer-controlled to perform their function in the right place at the right time in order to ensure that defective, and only defective, containers are rejected.

Obviously these complex systems are relatively sensitive to disturbances. Even the slightest displacement of optics, mirrors, illumination or positioning system, or the slightest asynchrony in the timing of spin motors, positioning, mirrors or scanning, may cause the inspection system to overlook a defective container, which from a safety and quality point of view is undesirable, or to reject good products, which from an economic and efficiency point of view is equally undesirable. As most of the above-mentioned components are mechanical, the risk of errors is not insignificant and has to be regarded. A speck on a lens from a bug, a blow to a mirror or optics by the cleaning staff, glass shards in a mechanical gear mechanism from a shattering container, etc., happen frequently.

In order to verify that an inspection machine really works as intended, i.e. from the consumer's point of view finds all the defective containers, and from the pharmaceuticals manufacturer's point of view does not reject good containers, a set of containers with known defects are typically run through the machine. This set consists for example of previously rejected containers that have subsequently been confirmed as in fact being defective. The set of known defects should preferably comprise containers with all the different kinds of defects in different sizes, etc., that the inspection machine manufacturer claims that the machine is able to detect, as well as a number of non-defective containers to test the false reject rate. If the inspection machine rejects the defective containers, and only the defective containers, within a certain tolerance percentage, the inspection machine can be said to function correctly. The set of known defects is used by the inspection machine manufacturers during development to improve the inspection stations and to verify that the inspection machine complies with regulatory tolerances, and it is used during production by the pharmaceuticals manufacturers to verify, e.g. between batches, if the inspection machine has unacceptable detection errors, which could e.g. be due to any of the above-mentioned events to the physical components within the machine.

A verification of an inspection machine by using the above-mentioned set of known defects is, however, not without disadvantages. The verification results are for example only probabilistic and general for the entire inspection machine, so when a test is done with an outcome of e.g. 23 rejects out of 100 containers of which 25 are known defects, it is hard to determine if the 2 non-detected defects are just the unavoidable, and to a certain extent acceptable, slips, or if they are in fact indicative of some physical error within the machine such as a mirror that has been misaligned by a few micrometers. Even if the verification rejected 25 containers, it would be uncertain whether they were the 25 defective containers or they were e.g. 23 defective containers and 2 false rejects - leaving 2 defective containers undetected in the pool of good containers.

Moreover, as the set of known defects is often just made up of confirmed rejects from previous, real batches of pharmaceuticals, and therefore are considered good test objects as they completely resemble the real products, they are however not possible to reproduce which makes it impossible to e.g. replace a shattered container or construct an identical test environment for other machines for reference, without having to expect different results. Further, when the containers from the set of known defects have to go through an inspection machine hundreds or thousands of times they get worn and often gain more and more defects such as scratches, or their original defects change with time. Altogether, sets of known defects compiled by simply collecting the rejected containers from the production of pharmaceuticals are not reliable, consistent or reproducible.

This problem has been sought solved by producing artificial sets of known defects, where the defects are considered reproducible. Such a set may e.g. comprise 100 containers with clear and absolutely clean water, where e.g. 25 of these are carefully prepared to include predetermined reference defects, e.g. containing steel or glass spheres with diameters ranging from 50 to 500 µm, containing shards or fibres, having cracks of specific lengths and widths at different positions and with different orientations, or having reference defects at their cap or bottom, etc. To the extent that artificial sets of known defects are possible to produce identically, they improve the possibility to compare results from different sets or to replace broken containers as compared to sets of real rejects. However, even the artificial sets of defectives get worn, and more importantly, they suffer from the same disadvantage of only providing a probabilistic and general verification result.

The above-described methods use a known defect matching the purpose of the particular inspection station being tested, e.g. a container with a particle when testing the particle detection. However, these methods only work with two-option, true or false, detections, and if the inspection station does not detect the known particle, it can only be derived that the inspection machine gives erroneous results on this particular test, but neither the reason, nor how close it was to be the correct result, can be provided. Also the statistical method has no control of the different test samples since these are fed into the machine in bulk, i.e. randomly.

A similar prior art disclosure comprises U.S. patent application publication No. 2005/0084974 to Veale et al., which regards headspace analysis, i.e. analysis of the gas contained in partially filled, sealed containers such as pharmaceutical vials, by using laser spectroscopy. The Veale et al. patent application discloses using reference vials with a known concentration of the gas being tested for, e.g. oxygen, or a known gas pressure if pressure is being tested, and using these reference vials to calibrate the measurement results for concentration measurements or pressure measurements, respectively, on product vials only. However, this method only works with numerical measurements, and it will only work as long as the amount of error, i.e. the adjustment value, stays within a reasonable range. This method does also not provide a reason for the error.

U.S. patent application publication No. 2010/0282650 A1 to Venaille discloses an inspection machine with reference containers that are each associated with an identifier and an expected result, thus making it possible to determine on a per-container level if the inspection machine performs well. It is further disclosed to process containers according to different settings of the inspection machine to compare and decide optimum settings.

### Summary of the invention

Hence, the inventors of the present invention has found that prior art test of inspection machines exposes the machine to the defect or reference the machine is supposed to detect, and does not provide a way to determine the reason for any error or inaccuracy, nor a way to determine how far from an erroneous result the inspection machine is, except when it comes to the single numerical measurements of gas concentrations or pressure.

In the light of the above, it is an object of an embodiment of the present invention to provide an inspection machine with a system test that enables reproducibility and which provides results that are specific and deterministic instead of general and probabilistic.

It is further an object of an embodiment of the present invention to provide a method for testing the status of an inspection machine in terms of the quality of the inspections stations and the existence of errors, preferably including individual subcomponents of inspection stations.

The invention relates to an inspection machine ASIM for inspecting containers CT such as vials according to claim 1.

By the present invention is provided an inspection machine enabling a test method which allows for reproducible, deterministic test results as opposed to the prior art statistical results. The test results from the present invention further allows for obtaining an indication of the amount of error or margin to an error, in general for many different kinds of inspection stations, sub-components and parameters as opposed to the prior art numerical gas concentration or pressure only offset detection. Even further, the test results from the present invention also allows for testing several kinds of parameters and inspection stations to provide an indication of the origin of a fault, e.g. a parameter of a sub-component, as opposed to prior art methods either merely stating that the entire machine is statistically faulty, or that for an unknown reason the numerical results have to be offset.

The above advantages mean that the present invention is used to monitor the individual status of the typically several mechanical, optical and electrical sub-components that are critical to the correct inspection results and which are prone to gradually or suddenly fail during use. This also comprises the components that provide the often critical timing for the inspections, for example ensuring that the vial is at the right place or having the right rotation or moving at the right speed at the time the inspection is performed.

In other words, the present invention allows for automatically monitoring the most error-prone bits and pieces inside a general inspection machine in a reproducible, deterministic and detailed way, which again allows for e.g. pointing to a specific faulty sub-component or predicting when a specific sub-component will fail.

According to the present invention, the above is obtained by implementing several physical standard objects, each designed to test one or more specific parameters of one or more specific sub-components. The standard objects of the present invention are not necessarily resembling a specific defect, e.g. an undesired particle, or a correct reference, e.g. a known gas concentration, as seen in the prior art, but go down into a higher level of detail to test sub-components and parameters. In other words, several of the standard objects are according to the invention not tested against the overall measurement or reject- or accept-algorithm by the inspection machine, but are treated by dedicated individual test algorithms, in the following called standard object analysis routines, to derive the detailed results. For example, a scratch detection station may according to prior art be expected to return a result like "defective" when a known defect is inspected. However, according to an embodiment of the present invention, the scratch detection station may e.g. return a result like "lens being displaced to the left" when one standard object of the present invention is inspected, and a result like "backlight illumination only 60% of normal" when another standard object of the present invention is inspected, and this detail is according to the invention obtained by the tailored design of standard objects and associated reference inspection results and standard object analysis routines.

The present invention improves several aspects of inspection machines such as error detection, fault finding, possibilities for performing tests at any time during a batch, etc., and moreover introduces several new features for inspection machines such as trend monitoring of the individual inspection stations or even specific parameters thereof, performing of partial tests targeted to specific parameters at any time, automatic adjustments or advising the operator based on deterministic test results, detailed comparison of different inspection machines and verification of conformity to specific regulations and thresholds due to the standard objects constituting actual references, etc.

These improvements and new features are made possible by the fact that the standard objects according to the present invention with their associated reference inspection results are both targeted to specific inspection stations or even specific parameters or sub-components thereof, e.g. optical focus or alignment, and at the same time are preferably completely reproducible. These properties make standard objects of the present invention actual reference test objects as opposed to the collected or prepared defective containers which have hitherto been used in the prior art as described above.

A standard object is according to an embodiment of the present invention configured with characteristics in such a way that the inspection station to which it is targeted will always come to a specific result, i.e. the reference inspection result associated with that standard object, when inspecting the standard object if and only if the inspection station or a specific targeted component, sub-component, of the inspection station is working properly.

In other words, the standard objects, or a number of them, are preferably targeted towards challenging sub-components of the inspection stations in order to determine a source of error, or a margin to a fault condition. Sub-components of inspection stations may e.g. comprise electronically controlled illumination, optics and mirrors related to the illumination, camera, line- or array-scanner, CCD or other image sampling device, possibly with related lenses, position controlled image tracking mechanisms, spin motors, preferably programmably controlled, for spinning vials and/or their content, etc., as well as format dependent settings and parameters of such sub-components.

For example, according to an embodiment of the invention, certain patterns of lines or graphical elements printed on a surface can be used to test e.g. focus and alignment properties of optical components of optical inspection stations with a deterministic result, and an object with known electrical properties, e.g. an electrical resistor, can be used to test e.g. high voltage leak detection inspection stations with a deterministic result. More details and further examples of embodiments of standard objects are given below in the detailed description.

The deterministic test results made possible by the present invention enables the control unit to determine information about the state of a specific inspection station or even a sub-component thereof, instead of only a general state of the entire inspection machine which is all that can be accomplished by prior art test methods. In other words, where the prior art methods were able to determine after having inspected e.g. 100 test containers that the entire inspection machine was generally all in all probably or probably not in good order, the present invention lets the inspection machine process e.g. 25 standard objects targeting different inspection stations and parameters thereof, and can thereafter determine the status of each of the inspection stations and even sub-components thereof, e.g. lenses, mirrors, etc.

A further aspect of the deterministic results made possible by the present invention is the possibility of obtaining more detailed information of the specific inspection stations. Whereas the present invention as described above generally enables the control unit to tell exactly what inspection station and possibly even which sub-component is flawed, a preferred embodiment of the invention further enables the control unit to measure, for at least for some kinds of inspection station components, the amount of error that is detected, i.e. providing a measure of the inaccuracies instead of simply a yes or no. For example, according to an embodiment of the invention, the comparison of a reference test result with the actual inspection result from an optical inspection station may provide information about how much the optics are out of focus or at what exact distance they are focused, instead of just determining that the optics are out of focus. Or, according to an embodiment of the invention, the comparison for an optical inspection station may provide information about how much and in which direction a mirror or sensor is misaligned, instead of just determining that it is misaligned.

Detailed error measurements as described above can in a very advantageous embodiment of the present invention be used for trend monitoring, automatic adjustments or simply useful and accurate advice to the operator - all of which are impossible to implement in a prior art inspection machine.

In an embodiment of the invention the control unit can directly use the detailed measurements to inform an operator or servicing staff e.g. which particular mirror is misaligned and how much it should moved or turned to work again.

In a more advanced embodiment the inspection machine components could be arranged with motors or other actuators to automatically carry out such adjustment steps as turning a mirror a few degrees or displacing a lens a few millimetres. Some adjustment steps can in an embodiment of the present invention be carried out entirely by software modifications. For example may a misalignment of a mirror in some embodiments be handled by e.g. adjusting an offset in the image processing software as long as the misalignment is small enough to let the entire container be within the capture area of the camera of the inspection station. Or the control unit may in an embodiment of the invention by software control be able to make the container stop for inspection at the position that fits the misaligned component, etc.

In an embodiment of the present invention, the detailed error measurements are analysed for trend monitoring. This means that the measurements are not only used for determining if they exceed a predetermined tolerance threshold, but also to estimate an indication of how close the good components may be to exceed the threshold and become erroneous components. The trend monitoring may be used for a number of purposes, e.g. to determine when to give the inspection machine the next major service, or to warn the operator that he should consider cleaning, repairing or calibrating a certain component at the first coming break in production, or that he should better order this or that specific spare part as it is probably going to be needed soon, or simply to inform the operator that everything is in good order and none of the slowly decaying components are about to start faulting. Trend monitoring is particularly useful and informative for components whose properties decay slowly, e.g. windows that slowly gets dustier, wearing parts that slowly changes their properties, e.g. springs, rubber parts or bearings, or components that displace themselves slowly due to vibrations, inaccurate resets or actuators, frequent impacts from the same direction, etc.

In an advantageous embodiment of the present invention the standard objects are further reproducible, i.e. they can be produced in high numbers and be reproduced at any time with substantially the exact same properties. For example, both of the above-mentioned types of standard object characteristics, i.e. high-resolution accurate graphical patterns and high quality electrical components with high precision properties, are reproducible. Most importantly the reproducibility makes it possible to simply obtain a new, identical standard object when an old one is ruined or worn out. The reference test result for that standard object will still be the same as before, and the machine will still be tested in the exact same way with the same tolerances as before, thereby avoiding all the disadvantages of replacing test containers in prior art test sets.

The reproducibility further improves the possibilities to compare different inspection machines, being it machines of the same type for control or machines of different brands for buying considerations. The present invention also enables a new and better way to administratively regulate the quality of inspection machines by e.g. requiring conformity to certain tolerances defined with respect to the references, e.g. standard objects of the present invention. This has hitherto not been possible, as actual, reproducible references with detailed predetermined reference test results have not been available before.

An advantageous embodiment of the present invention is obtained when said inspection station or control unit is arranged to analyse said standard object-dependent inspection results related to the standard object and obtained from the at least one of the one or more inspection stations.

An advantageous embodiment of the present invention is obtained when said inspection station or control unit is arranged to compare said standard object-dependent inspection results with reference inspection results; the reference inspection results being predetermined for different combinations of standard objects, inspection stations and standard object analysis routines.

An advantageous embodiment of the present invention is obtained when one or more of said reference inspection results are multi-dimensional, e.g. a picture, or multi-variable, e.g. comprising several parameters.

According to the present invention said standard objects and said standard object analysis routines are arranged to establish standard object-dependent inspection results that represent status information about sub-components of said inspection stations.

An advantageous embodiment of the present invention is obtained when said standard objects and said standard object analysis routines are arranged to establish standard object-dependent inspection results that represent fault margin information.

An advantageous embodiment of the present invention is obtained when said standard objects and said standard object analysis routines are arranged to establish at least one standard object-dependent inspection result that represent mechanical parameters of said inspection machine.

An advantageous embodiment of the present invention is obtained when said standard objects and said standard object analysis routines are arranged to establish at least one standard object-dependent inspection result that represent optical parameters of said inspection machine.

An advantageous embodiment of the present invention is obtained when said standard objects and said standard object analysis routines are arranged to establish at least one standard object-dependent inspection result that represents electrical or electronic data parameters of said inspection machine.

An advantageous embodiment of the present invention is obtained when said standard objects and said standard object analysis routines are arranged to establish at least one standard object-dependent inspection result that represents timing parameters of said inspection machine.

An advantageous embodiment of the present invention is obtained when at least one of the standard objects is arranged with one or more specific characteristics that corresponds to one or more specific test parameters that are specifically tested by one or more of the inspection stations, and wherein one or more of the standard objects are selected from a list comprising: light distribution standard objects LDSO, focus standard objects FCSO, ALSO, alignment standard objects ALSO, unfolding standard objects UFSO, high voltage standard objects HVSO, enlargement standard objects, spin speed standard objects, and timing standard objects.

In an advantageous embodiment of the present invention, the system test arrangement comprises a standard object storage SSO for holding the standard objects. In a preferred embodiment the standard object storage holds standard objects in a way compatible with the conveying system and other handling mechanisms present in the inspection machine. In a preferred embodiment the standard object storage with its standard objects may be easily exchangeable so as to easily reconfigure the system test arrangement when the inspection machine is reconfigured for a different kind of containers which requires different standard objects, e.g. due to physical size or shape.

In an advantageous embodiment of the present invention the inspection machine comprises a conveying system IF, EW, SW1, IW, TW1, MIC, TW2, CV1, SW2, CV2 for transporting the containers to the inspection stations and the sorter; and wherein the standard object storage SSO is arranged in connection with the conveying system so as to facilitate the conveying system to also transport the standard objects to the inspection stations. By facilitating insertion and transport of the standard objects by the inspection machine's present conveying system or other mechanical handling system the reconfiguration of an inspection machine to allow for testing with standard objects according to the present invention may be performed quickly and easily without major changes to the inspection machine.

An advantageous embodiment of the present invention is obtained when the system test arrangement comprises at least two standard objects; wherein the control unit is arranged to control an exposure of at least one of said at least two standard objects to at least one of said one or more inspection stations, and wherein said at least two standard objects are arranged in a predetermined sequence to facilitate the control unit to control or at least track the movement of the individual standard objects.

An advantageous embodiment of the present invention is obtained when the at least one standard object comprises a machine readable identification to facilitate the one or more inspection stations or the control unit to identify the standard objects.

The invention further relates to a method for testing the performance level of an inspection machine ASIM comprising one or more inspection stations IS 1 - ISn for inspecting containers CT, such as vials, according to claim 9.

An advantageous embodiment of the present invention is obtained when the method comprises comparing the standard object-dependent inspection results with predetermined reference inspection results and basing the inspection machine status on the basis of a result of the comparison.

An advantageous embodiment of the present invention is obtained when the reference inspection results are predetermined for different combinations of standard objects, inspection stations and standard object analysis routines.

An advantageous embodiment of the present invention is obtained when the inspection machine status represents information about sub-components of said inspection stations.

An advantageous embodiment of the present invention is obtained when the inspection machine status represents fault margin information for inspection stations or sub-components thereof.

An advantageous embodiment of the present invention is obtained when the inspection machine status represents mechanical parameters of said inspection machine.

An advantageous embodiment of the present invention is obtained when the inspection machine status represents optical parameters of said inspection machine.

An advantageous embodiment of the present invention is obtained when the inspection machine status represents electrical or electronic data parameters of said inspection machine.

An advantageous embodiment of the present invention is obtained when the method comprises providing information for establishing correction guidance on the basis of said inspection machine status.

An advantageous embodiment of the present invention is obtained when the method comprises providing information for correcting an error or inaccuracy on the basis of said inspection machine status.

An advantageous embodiment of the present invention is obtained when the step of providing one or more standard objects comprises providing at least two standard objects and sequence information to facilitate standard object inspection routines and standard object-dependent results.

An advantageous test method is obtained when the step of providing the standard objects for inspection involves conveying the standard objects from a standard object storage SSO to the inspection stations.

A further advantageous embodiment is obtained when the standard objects are conveyed back to the standard object storage after one or more standard object-dependent inspection results have been obtained by the inspection stations. Thereby the standard objects are readily available for reuse.

An example not covered by the claims relates to a system test arrangement SSO, SO, SW3, ECU for use in connection with an inspection machine ASIM comprising one or more inspection stations IS1 - ISn for inspecting containers CT such as vials, the system test arrangement comprising one or more standard objects SO; HVSO; LDSO; FCSO; ALSO; UFSO and for each standard object an associated reference inspection result for one or more of the inspection stations of the inspection machine, wherein the standard objects have one or more specific characteristics that are arranged to challenge specific sub-components of the one or more inspection stations..

When the system test arrangement comprises a standard objects storage SSO and a mechanical handling equipment SW3 arranged to be able to transfer the standard objects from the standard objects storage to a mechanical handling equipment IF, EW, SW1, IW, TW1, MIC, TW2, CV1, SW2, CV2 of the inspection machine and to receive the standard objects from a mechanical handling equipment of the inspection machine and return them to the standard objects storage, an advantageous embodiment of the present invention is obtained.

The invention further relates to a set of standard objects for use in test of an inspection machine ASIM comprising one or more inspection stations IS 1 - ISn for inspecting containers CT such as vials according to claim 14.

In an advantageous embodiment of the present invention, the standard objects are selected from a list comprising light distribution standard objects LDSO, focus standard objects FCSO, ALSO, alignment standard objects ALSO, unfolding standard objects UFSO, high voltage standard objects HVSO, enlargement standard objects, spin speed standard objects and timing standard objects. It is noted that embodiments of the present invention may comprise other standard objects or parts or combinations thereof.

The invention further relates to a system test arrangement for use with an inspection machine comprising one or more inspection station according to claim 15.

### The drawings

The invention will in the following be described with reference to the drawings where
fig. 1 illustrates a prior art inspection machine,
fig. 2 illustrates an inspection machine with a system test arrangement according to an embodiment of the present invention,
fig. 3 illustrates an embodiment of a high voltage standard object according to an embodiment of the present invention,
fig. 4 illustrates an embodiment of a light distribution standard object according to an embodiment of the present invention,
fig. 5 illustrates an embodiment of a focus and contrast standard object according to an embodiment of the present invention,
fig. 6 illustrates an embodiment of an alignment standard object according to an embodiment of the present invention,
fig. 7 illustrates an embodiment of a unfolding standard object according to an embodiment of the present invention,
fig. 8A - 8I illustrate graphical patterns and elements according to embodiments of the present invention,
fig. 9A - 9H illustrate configurations of standard objects according to an embodiment of the present invention, and
fig. 10 - 13 illustrate different embodiments of an inspection machine according to the present invention.

### Detailed description

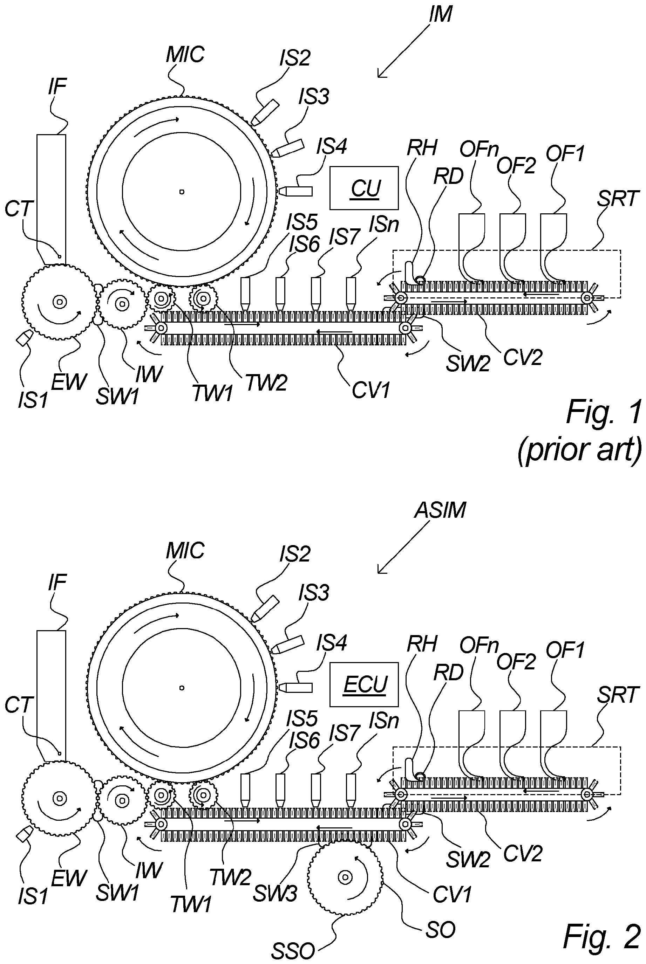

In order to aid the description of the different aspects and embodiments of the invention, an example of a prior art inspection machine for inspecting containers, vials, ampoules, syringes and the like, is provided in figure 1. It is noted that the construction in figure 1 is only one example of inspection machines. Numerous ways to build inspection machines exist, and the present invention is not limited to a certain inspection machine but can be used to improve any inspection machine in the art. It is further noted, that because of the invention's much broader appeal the following description is more a principle description than it is a specification of the illustrated machine. Hence, the wording used should also be abstracted from any particular, narrow technical meaning possibly conveyed within a particular field, and merely be seen as a general description of elements of an example inspection machine provided only for illustrative purposes.

Figure 1 illustrates an inspection machine IM as seen from above. Without prejudice to the present invention, the particular machine in this example is for inspecting oblong, substantially cylindrical, containers while maintaining them in a vertical position at all times.

The inspection machine IM comprises a number of inspection stations IS1 - IS7, ISn. The inspection stations are located along a container travelling path so that the containers pass by the inspection stations on their way through the inspection machine, as described in the following. The inspection stations in figure 1 are illustrated as identical and simple for generalisation and to not complicate the drawing, but actually the inspection stations will typically look quite different due to the different areas of the containers that they are inspecting and due to various technologies used for different tests.

With regard to the container travelling path, the containers are first transported to an in-feed IF of any suitable kind, either manually or by upstream conveying means. To not obscure the drawing, only a single container CT is illustrated at the in-feed IF in figure 1, and no further containers are illustrated along the container travelling path. Obviously the in-feed would in a typical situation hold plenty of containers, as would most of the elements along the travelling path. From the in-feed, the containers are transferred to a conveying system for transporting them through the inspection machine. In the example of figure 1, the conveying system comprises an entry wheel EW, an intermediate wheel IW, two transfer wheels TW1, TW2, a main inspection carousel MIC and two clip conveyors CV1, CV2. The wheels have indentations around their circumferences to receive and hold the containers.

In figure 1 the main route for the containers is to be received from the in-feed IF by the entry wheel EW where a coarse inspection of major chips and cracks may be carried out by the first inspection station IS1 to not bring heavily damaged containers forward into the machine. Then the containers are transferred to the intermediate wheel IW, then onto the first transfer wheel TW1 and then to the main inspection carousel MIC. In the example of figure 1, the main inspection carousel holds the containers at their top and bottom so that they can be spun around a vertical rotation axis, whereas the other transportation means typically hold the containers by means of clips, thus making it possible to inspect the top and bottom. During the trip around the main inspection carousel MIC the containers are in this example put to test in three inspection stations IS2 - IS 4. When reaching the second transfer wheel TW2 the containers are thereby transferred to the first clip conveyor CV1, which allows for a linear movement past e.g. four further inspection stations IS5 - ISn. At the end of the first clip conveyor, they are transferred to the second clip conveyor CV2, which carries the containers through a sorting area SRT where the containers are diverted into a number of suitable out-feeders OF1, OF2, OFn, or eventually to a reject dump by means of a reject hook in this example. A central control unit CU processes the inspection results and controls the sorting in the sorting area SRT.

Furthermore, the control unit is able to cause a container to be returned to the inspection stations if for example the inspection results are unclear or need to be verified. In that case, a switch SW2 will cause the container to stay at the first conveyor CV1 instead of being transferred to the second conveyor CV2. When the container gets to the first end of the first conveyor CV1, it is transferred to the first transfer wheel TW1 and onto the main inspection carousel MIC. In order to ensure that there is an empty clip position in the transfer wheel TW1, a switch SW1 is provided to create empty positions on the intermediate wheel IW and thus indirectly on the first transfer wheel TW1.

Figure 2 illustrates an automatic standards inspection machine ASIM according to an embodiment of the present invention. The inspection machine comprises all the same elements as the inspection machine IM in figure 1 described above. In addition, the automatic standards inspection machine ASIM comprises a storage for standard objects SSO as well as a third switch SW3 to transfer standard objects between the storage for standard objects SSO and the first conveyor CV1 in both directions. In figure 2 is simply illustrated one standard object SO in the storage SSO, but typically several, e.g. 10 - 25 standard objects will be included. The automatic standards inspection machine ASIM also comprises an extended control unit ECU, which beside what is mentioned about the control unit above, also is arranged to control the transfer of standard objects to and from the inspection stations, and to process the inspection results of standard objects.

In a typical setup according to an embodiment of the present invention, the standard objects will under control of the extended control unit at certain intervals or triggered by certain events be transferred along the container travelling path and be inspected by the inspection stations preferably exactly as the containers CT. They are just being delivered to and from the storage SSO instead of from the in-feed to the second conveyor CV2 and the sorter SRT. This alternative travelling through the machine is enabled by controlling the first switch SW1 to create empty positions and the second switch SW2 to let the standard objects stay on the first conveyor CV1.

The extended control unit ECU, or an associated database or other suitable data storage, also holds reference inspection results to compare with the inspection results obtained by the inspection stations when inspecting the standard objects. Depending on the specific inspection station technologies used, the specific standard objects used, and the parameters that are tested for, there may be several reference inspection results associated with different combinations of standard object and inspection station.

In a preferred embodiment of the invention, an inspection station has a container analysis routine which it carries out for each container that comes by, e.g. the container analysis routine for a particle detection station may comprise taking a picture and analysing the image for foreign particles. But the inspection station also has one or more standard object analysis routines to use when standard objects come by. For example the particle detection station may perform a first standard object analysis routine comprising taking a picture and analysing the image for light distribution when a backlight testing standard object comes by, and perform a second standard object analysis routine comprising taking a picture and determining the location of a graphic object in the image when an alignment testing standard object comes by.

In other words, the standard objects, or a number of them, are preferably targeted towards challenging sub-components of the inspection stations in order to determine a source of error, or a margin to a fault condition. Sub-components of inspection stations may e.g. comprise electronically controlled illumination, optics and mirrors related to the illumination, camera, line- or array-scanner, CCD or other image sampling device, possibly with related lenses, position controlled image tracking mechanisms, spin motors, preferably programmably controlled, for spinning vials and/or their content, etc., as well as format dependent settings and parameters of such sub-components.

For example, the third inspection station IS3 may be expected to produce a certain inspection result when submitted to a certain standard object, and a reference inspection result is thus saved for testing that combination. The same standard object may cause a completely different, yet expectable, result at e.g. inspection station six, and a reference inspection result is therefore also saved for that combination. At inspection station 7, for example, the inspection result for that particular container may be irrelevant or less indicative for the quality of inspection station 7, and a reference inspection result may therefore be excluded for that combination.

The different inspection stations are typically configured very differently in terms of what part of the containers they are inspecting and how they conduct that inspection. This means that use of different properties and features is necessary to test different inspection stations. One inspection station may e.g. be tested with a certain graphical pattern at the upper part of the object, another with a completely clear solid glass cylinder at most of its height, and yet a third with a specific electrical resistance. A set of standard objects may therefore contain as many standard objects as there are inspection station parameters that needs to be tested.

However, in a preferred embodiment some standard objects may contain characteristics, e.g. a certain graphical pattern, that is carefully designed so that two or more inspection station parameters can be tested by a single standard object. This could e.g. be a standard object having a graphical pattern that allows measuring of both focus and alignment of an inspection station for finding particles in the container content and also having a certain cap configuration allowing measuring of parameters of a cap inspection machine.

With standard objects of this type which are used to measure several parameters of possibly several inspection machines, they should each be associated with as many reference inspection results as they have measurement purposes.

For example, in an embodiment of the invention, a set of standard objects may e.g. comprise 5 standard objects for testing properties of the inspection stations that utilizes high voltage leak detection (HVLD) and 10 standard objects for testing properties of the inspection stations that utilizes optical units. In general all of the standard objects should preferably comprise the spatial features required to be compatible with the conveyor system used in the relevant inspection machine. Hence, if the inspection machine at some points uses clips at the middle part of the containers to inspect the top and bottom, and at other points supports the top and bottom of the containers to inspect the middle part, the standard objects should at their top, middle and bottom comprise a shape that works with the clips and supports. In an inspection machine where only the bottom of the containers are supported, e.g. by standing on a conveyor band, there are no further requirements to the standard objects than that they have to be able to stand and be able to pass through any narrow passages through the inspection machine.

The e.g. 5 standard objects for HVLD testing may e.g. comprise cylinder shaped objects of non-conductive material, e.g. plastic, with electrical resistors, complex impedances or capacitors implanted to give a predetermined electrical response to the high voltage applied by the HVLD stations. The electrical components, e.g. resistors should preferably be selected so that the HVLD stations measures an unexpected electrical response if the HVLD station applies a wrong voltage or if its probes are not where they are supposed to be with respect to the standard object. The standard objects could also be made of a semiconductive material, comprise a coating of certain conductivity, have certain capacitive properties, etc. The e.g. 5 standard objects targeting the HVLD stations may preferably comprise standards for testing an upper leak detector, a lower leak detector, a top leak detector, a low filling detector and a capacitive body profile verification station. In a preferred embodiment the characteristics of some or all of the above-mentioned e.g. 5 HVLD standard objects may be combined into fewer than 5 standard objects, such that all e.g. 5 purposes are tested by only e.g. 3 standard objects.

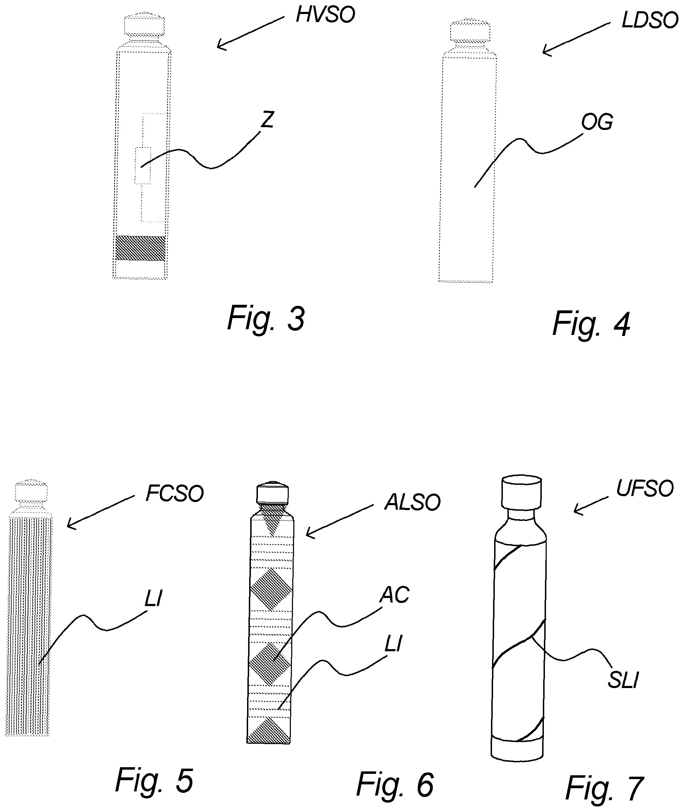

Figure 3 illustrates an example of a standard object HVSO which is arranged with electrical impedance Z, e.g. a resistor, so as to provide the standard object HVSO with a predetermined reference electrical response when a high voltage is applied at the top and bottom of the container by a HVLD inspection station. The analysis routine used by a HVLD inspection station when exposed to this standard object is preferably according to the invention different that the container analysis routine normally applied by the HVLD inspection station. In this particular example, the standard object analysis routine may comprise analyzing the inspection result to determine what voltage the inspection machine is applying, if the probe locations and distance to the standard object are correct, and it may even for example cause a special voltage sweep to be applied to the standard object for different test purposes.

The e.g. 10 standard objects for optical station testing may e.g. comprise components with certain graphical patterns, colors, shapes, opacity, etc. which can be used to test and verify different kinds of optical measurement and support units in the different inspection stations.

For example may the light profile of the so-called backlight used to illuminate the containers from a direction opposite the camera on certain inspection stations as well as the optical noise of those stations be tested by letting the camera capture an image of a standard object comprising a water filled container or a pure solid glass cylinder and comparing the captured image or characteristics thereof with a reference image or reference characteristics. As mentioned above, in a preferred embodiment this example constitutes a special standard object analysis routine different from the container analysis routine normally applied by these certain inspection stations.

The light profile and optical noise of stations with e.g. illumination at the side or front of the containers may be tested with e.g. a container made of or containing a translucent material with a light distributing effect, e.g. a cylinder made of solid so-called milk or opal glass, e.g. a glass with added tin-dioxide or components of calcium and phosphate, or other colouring or opacifying components. When light is illuminating the light distributing material from the side or the front relative to the direction of an observer, e.g. a camera of an inspection station, the standard object will obtain a characteristic appearance determined by the angle, brightness and spreading of the illumination. If a snapshot captured by the camera differs from the expected appearance with regards to light distribution it can be concluded that the illumination system is flawed, and often the way of difference tells what is wrong with the illumination, e.g. the direction, spread or brightness. As mentioned above, in a preferred embodiment this example constitutes a special standard object analysis routine different from the container analysis routine normally applied by these certain inspection stations.

Figure 4 illustrates an example of a standard object LDSO comprising a solid body of an opal white material OG, e.g. so-called milk glass, which is useful for testing the illumination at certain inspection stations.

Focus, contrast and tracking, i.e. correct alignment, of cameras and optics may be tested with containers or cylinders comprising graphical elements or components of certain shapes. An optical inspection station is expected to produce a certain reference output when capturing an image or performing other optical measurements of the graphical elements or shaped components of a standard object of this kind, and any deviance of the output from the expected reference output may be used to determine which optical element is defective and in which way.

For example may a number of narrow black lines on a white or light background be used to test focus and contrast as a defect in this regard will cause a captured image to not comprise a clear edge between black pixels caused by the lines and white or light pixels caused by the background. In an advanced embodiment, the kind and degree of blurring of the edges of lines or graphical elements of different shapes, orientation and location, may be analysed to determine what the cause of the defect is.

Focus and alignment may for example be tested by a component with a shape that differs with relation to its distance from e.g. the vertical rotation axis of the standard object, and/or with relation to the vertical center, ends or other predefined position at the standard object. An image captured by an inspection station may then be analysed to find the point(s) at the shaped component where focus is best, typically determined by e.g. finding the area in the image with the highest average contrast between pixels. As the component shape and location in the standard object is known, the distance between the focus point(s) and e.g. the rotation axis, the top or bottom, etc., can be determined, which in turn can be used to determine the focal point relative to the optics of the inspection station and thus evaluate if focus is correct. Also because of the varying shape of the component, the image can be analysed to determine if the component is correctly positioned in the image, e.g. simply by correlating the captured image with a previously captured reference image, or by performing image recognition to find the component in the image and determine its vertical and horizontal position relative to the optics. As the position of the component in the standard object is known and as the analysis gives the position of the component relative to the optics, it can be determined if the alignment of the standard object with the optics is correct. The analysis results regarding focus and alignment may be used in a very detailed error report possibly including precise instructions for an operator on how to adjust the optics to correct the defect or even, in an advanced embodiment with controllable optics, the system may correct itself by adjusting the optics on the basis of the analysis results. In preferred embodiments these examples constitute standard object analysis routines different from the container analysis routines normally applied by these certain inspection stations.

Figure 5 illustrates an embodiment of a focus and contrast testing standard object FCSO comprising a cylindrical body with several narrow, black lines LI printed on it. It is noted that the lines or other well-defined, contrast-rich graphics may be printed in other orientations or patterns as well within the scope of the present inventinon.

Figure 6 illustrates an embodiment of a focus and alignment testing standard object ALSO comprising a combination of horizontal bars or lines LI for testing focus in the same way as described above with regard to figure 5, and a number of solid cones AC for testing alignment and possibly advanced focus as described above. The horizontal bars may be printed on the container body, or may be comprised of discs inserted in the body. The solid cones comprise components fixed inside the container body. Cones are a preferred shape as their two-dimensional representation e.g. in an image captured by an inspection station, does not change when the standard object is rotated around a vertical axis, and neither do their indication of center represented by the cone apex or their horizontal position represented by their cone base move during such rotation. It is noted that other components with other shapes or different position, or combined with different graphical elements can be used instead of the cones for tracking, and that such are within the scope of the present invention.

Figure 7 illustrates an embodiment of a standard object arranged to test a so-called unfold inspection station where a container is captured several times e.g. by means of a line scanner, while it rotates or its contents rotates, so that all the captured lines together form an image which represents an unfolded view of the cylindrical container or contents. The unfold standard object UFSO comprises a container-like body where a straight line SLI has been printed while the body rotates so it forms a spiral up the body of the standard object. When an unfolding inspection station captures and unfolds an image of the unfold standard object UFSO, it should result in a straight, diagonal line if the unfolding inspection station is not defective. It is noted that variants or other ways of testing an unfolding inspection station is within the scope of the present invention, too. In preferred embodiments these examples constitute standard object analysis routines different from the container analysis routines normally applied by these certain inspection stations.

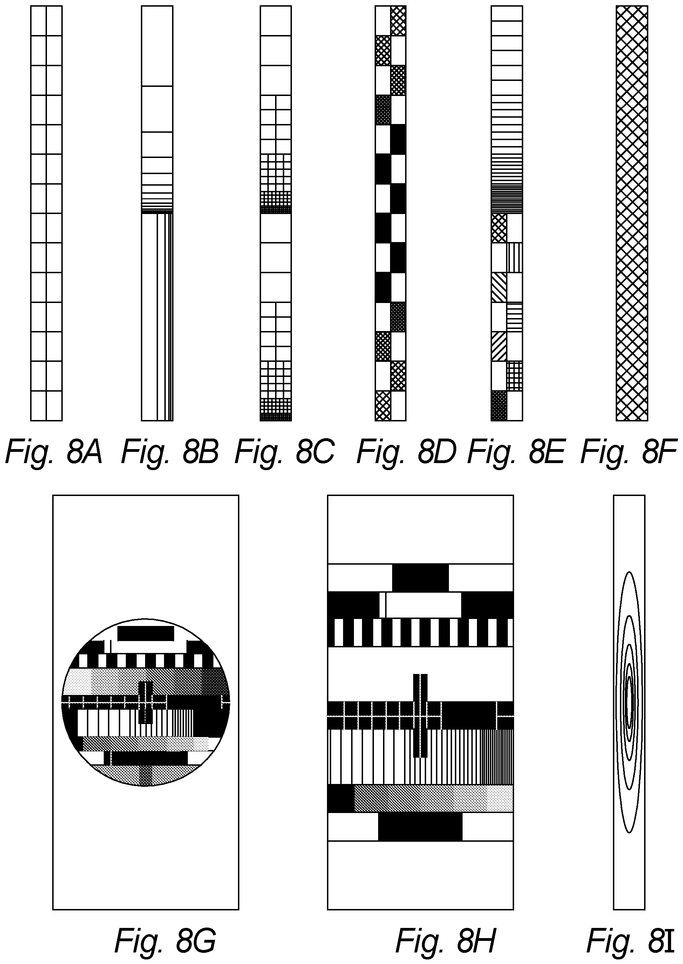

Figures 8A - 8I illustrate variants of graphical patterns that can be used to test different optical properties of an inspection station such as focus, contrast, alignment, etc., in alternative embodiments of the present invention. It is noted that some inspection stations may utilize color sensors, color cameras and/or evaluation algorithms that takes colors into account, e.g. to be able to verify cap or plunger colors for coding purposes, or in applications where discoloration of cap, container, plunger or the pharmaceutical contents themselves may indicate a defect container. By using different coloring of lines or areas, e.g. as known by television test charts, also the color capturing and evaluation of the inspection stations can be tested. In particular, figures 8G and 8H may comprise colored fields, possibly in combination with grayscale fields, according to an embodiment of the present invention.

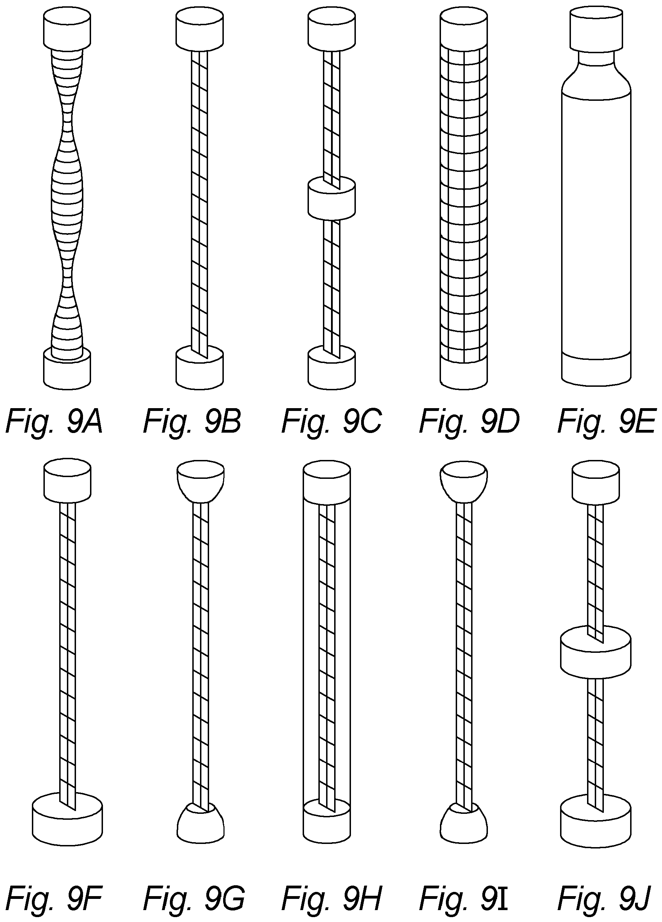

Figures 9A - 9H illustrate different shapes and configurations of standard objects according to different embodiments of the present invention. The shown patterns are for illustration purposes only; they may be substituted for any of the patterns and/or components described and illustrated herein, or variants thereof. As mentioned above, the standard objects for a particular inspection machine should be compatible with the conveying system of that machine. Hence, for an inspection machine using conveyors with clips holding the containers at their body at some times, the embodiments of e.g. figures 9B, 9F, 9G and 9I may probably not be relevant, whereas all of the illustrated embodiments may be used in a conveying system only holding the containers at their top and/or bottom. The embodiment in figure 9A provides some of the same features as the focus and alignment standard object illustrated in figure 6 in a different way. Figures 9B, 9D and 9H illustrate that graphical elements may either be printed on the body as in figure 9D, on an alternative surface as illustrated in figure 9B, or on an alternative surface comprised inside the body as in figure 9H.

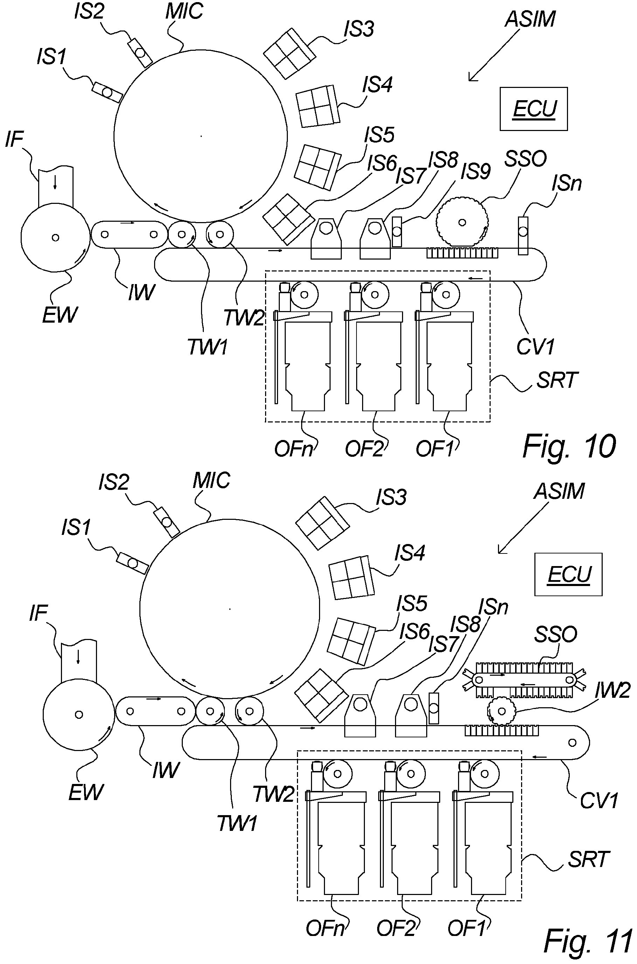

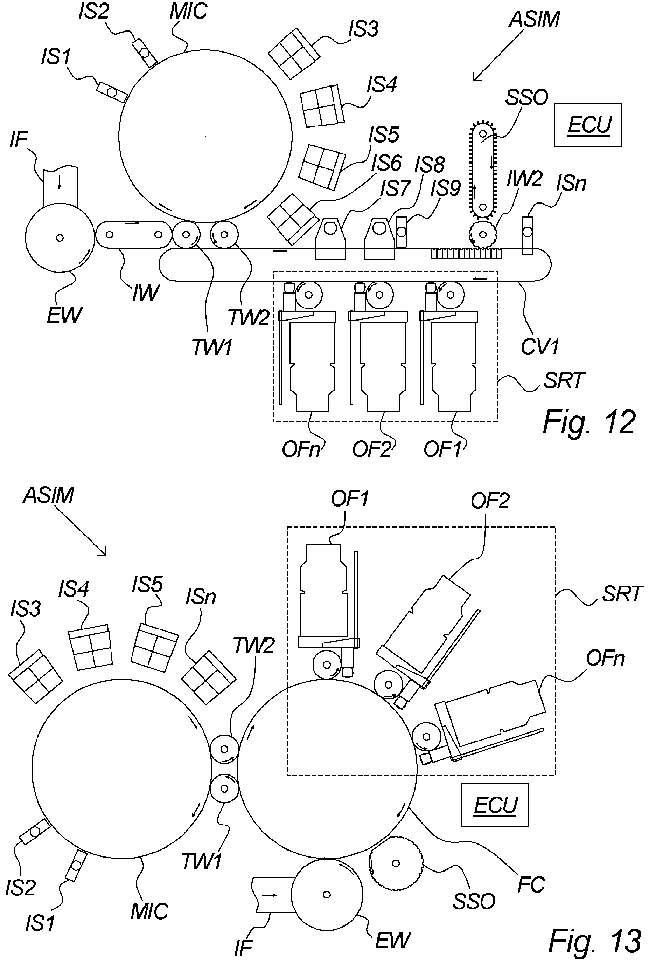

Fig. 10 - 13 show different alternative embodiments of inspection machines according to the present invention to illustrate that the particular configuration of the inspection machine is generally flexible within the scope of the invention, and several other configurations can be derived within the invention. In fig. 10 - 13 generally the same reference signs have been used as used above for fig. 1 and 2 for generally the same elements. In fig. 10 - 13 the container clips, wheel indentations for holding containers, etc. have for simplicity generally not been drawn in the figures except for the standard object storage SSO in fig. 10 - 13, and its intermediate wheel IW2 in fig. 11 and 12. A few container clips are included for illustration purposes on the clip conveyor CV1 in fig. 10 - 12. However, the wheels, conveyors, etc. of fig. 10 - 13 comprise or cooperate with means for receiving and holding containers. Also not shown in fig. 10 - 13 for simplification are the switches or other control means used to control the transfer of a container from one wheel or conveyor to an adjacent wheel or conveyor. In preferred embodiments of the invention such switches controllable by the control unit ECU are arranged at all places where wheels, conveyors, carrousels, etc., have contact or near contact in fig. 10 - 13.

The different embodiments in fig. 10 - 13 have different numbers of inspection stations IS1 - IS9, ISn, and the inspection stations are drawn in different ways. These differences serve the purpose of illustrating that different possibilities exists, and can be combined in any way, and embodiments with fewer or even more inspection stations, or inspections stations with any other shape are within the scope of the invention. The same applies to the number and type of outfeeders OF1, OF2, OFn. The drawings in fig. 10 - 13 do not show a reject dump or reject hook, but this could obviously be implemented as in fig. 2, or one of the outfeeders OF1, OF2, OFn could be arranged to serve as reject dump, in any of the embodiments of the invention.

In all of the embodiments in fig. 10 - 13 a standard object storage SSO is provided for holding standard objects SO when not being tested in the machine, and the standard object storage SSO is located so that standard objects can be transferred to a conveyor, wheel or carrousel of the inspection machine and back to the standard object storage. In fig. 11 and 12 are shown that a intermediate wheel IW2 may be provided between the standard object storages SSO and the e.g. clip conveyor CV1 where the standard objects are transferred to, and such intermediate wheels or other intermediate conveying systems may be arranged in any embodiment within the scope of the inventin. The configuration of a standard object storage SSO may e.g. be selected according to the number of standard objects to be stored therein, the physical configuration of the standard objects, and/or the degree of freedom in controlling which standard object should be released to the machine next.

In fig. 13 the linear conveying system for the sorting and outfeeding shown e.g. in the embodiments of fig. 2 and 10 - 12, has been substituted for a feed carrousel FC which in this embodiment also receives containers from the infeed IF via the entry wheel EW. In other embodiments of the invention the two or more carrousels may be combined with linear conveyor systems, or configured differently.

In preferred embodiments of the invention, the conveyor systems, wheels and carrousels are combined and configured in a way that allows a container or standard object to get round and back to inspection stations even when having passed them all for a second consideration, and further the system should preferably be configure to allow standard objects to travel from the standard object storage SSO past the inspection stations and back to the standard object storage for enabling automatic dispatch of standard objects into the machine and automatic reloading the storage to be ready for the next tests.

In preferred embodiments of the invention the standard objects SO are stored in the standard object storage SSO in such a way that the control unit ECU knows or is able to determine their individual sequence or locations. Thereby the control unit may be able to control that a standard object with a specific purpose, e.g. testing the backlight of the particle detector station, can be dispatched when desired, e.g. upon operator request or if the reject ratio of polluted containers increases. Also for the purpose of knowing when the object being inspected in a certain inspection station is in fact a standard object and not just one of the container, it is relevant that the control unit keeps track of the standard objects and can control the inspection station to use the standard object analysis routine corresponding to the particular standard object instead of the normal container analysis routine. The tracking of the standard objects can be obtained by storing them in a predetermined sequence, or the standard objects may comprise machine readable identification, e.g. printed on the objects or using near field communication technologies. According to the invention the machine readable identification includes standard object analysis routines, or identifies a source for that information. In an example which is not part of the claims the machine readable identification may include relevant standard-object dependent reference inspection results. The above-described tracking and/or identification of standard objects may advantageously be implemented in any embodiment of the present invention.

In the various embodiments of the present invention, the control unit ECU may within the scope of the invention either be a central processor or be distributed among different processors, e.g. in each inspection station, etc., or a combination thereof. The analysis routines performed by the inspection stations to establish an inspection result may likewise in the different embodiments of the invention be performed by local processors at the inspection stations or by outsourcing the job to a central processor, which could be the control unit ECU or a special analysis processor.

## Claims

1. An inspection machine (ASIM) for inspecting containers (CT) such as vials, said inspection machine (ASIM) comprising:
one or more inspection stations (IS1 - ISn) comprising sub-components from the list of mechanical, optical and electrical sub-components, each inspection station (IS 1 - ISn) being arranged to perform a container analysis routine for inspecting a container (CT) and to establish a container-dependent inspection result;
a control unit (ECU) arranged to analyse said container-dependent inspection results related to the container (CT) and obtained from the one or more inspection stations (IS1 - ISn), and on the basis thereof determine if the container (CT) fulfils one or more grounds for rejection; and
a sorter (SRT) at least partly controlled by the control unit (ECU) to divert the container (CT) if the control unit (ECU) has determined that the container (CT) fulfils one or more grounds for rejection;
**characterised in that** the inspection machine (ASIM) further comprises a system test arrangement (SSO, SO, SW3, ECU) in connection with the one or more inspection stations (IS1 - ISn), the system test arrangement (SSO, SO, SW3, ECU) comprising at least one standard object (SO; HVSO; LDSO; FCSO; ALSO; UFSO) having one or more specific characteristics that are arranged to test specific of said sub-components of the one or more inspection stations (IS1 - ISn);
wherein at least one of the one or more inspection stations (IS1 - ISn) are further arranged to perform a standard object analysis routine, which is different from the container analysis routine, and which is a dedicated individual test algorithm for inspecting a standard object (SO; HVSO; LDSO; FCSO; ALSO; UFSO) and to establish a standard object-dependent inspection result; said standard objects (SO; HVSO; LDSO; FCSO; ALSO; UFSO) and said standard object analysis routines being arranged to establish standard object-dependent inspection results that represent status information about said sub-components of said inspection stations (IS 1 - ISn)s; and
wherein said inspection station (IS1 - ISn) or control unit (ECU) is arranged to analyse said standard object-dependent inspection results related to the standard object (SO; HVSO; LDSO; FCSO; ALSO; UFSO) and obtained from the at least one of the one or more inspection stations (IS1 - ISn).

2. The inspection machine (ASIM) according to claim 1, wherein said inspection station (IS1 - ISn) or control unit (ECU) is arranged to compare said standard object-dependent inspection results with reference inspection results; the reference inspection results being predetermined for different combinations of standard objects (SO; HVSO; LDSO; FCSO; ALSO; UFSO), inspection stations (IS1 - ISn) and standard object analysis routines.

3. The inspection machine (ASIM) according to claim 2, wherein one or more of said reference inspection results are multi-dimensional, e.g. a picture, or multi-variable, e.g. comprising several parameters.

4. The inspection machine (ASIM) according to any of the claims 1 to 3, wherein said standard objects (SO; HVSO; LDSO; FCSO; ALSO; UFSO) and said standard object analysis routines are arranged to establish standard object-dependent inspection results that represent fault margin information, or at least one standard object-dependent inspection result that represents one or more of mechanical parameters, optical parameters, electrical or electronic data parameters and timing parameters of said inspection machine (ASIM).

5. The inspection machine (ASIM) according to any of the claims 1 to 4, wherein at least one of the standard objects (SO; HVSO; LDSO; FCSO; ALSO; UFSO) is arranged with one or more specific characteristics that corresponds to one or more specific test parameters that are specifically tested by one or more of the inspection stations (IS1 - ISn), and wherein one or more of the standard objects (SO; HVSO; LDSO; FCSO; ALSO; UFSO) are selected from a list comprising:
- light distribution standard objects (LDSO),
- focus standard objects (FCSO, ALSO),
- alignment standard objects (ALSO),
- unfolding standard objects (UFSO),
- high voltage standard objects (HVSO),
- enlargement standard objects,
- spin speed standard objects, and
- timing standard objects.

6. The inspection machine (ASIM) according to any of the claims 1 to 5, wherein the inspection machine (ASIM) comprises a conveying system (IF, EW, SW1, IW, TW1, MIC, TW2, CV1, SW2, CV2) for transporting the containers (CT) to the inspection stations (IS 1 - ISn) and the sorter (SRT); and wherein said system test arrangement (SSO, SO, SW3, ECU) comprises a standard object storage (SSO) for holding the standard objects (SO; HVSO; LDSO; FCSO; ALSO; UFSO) and arranged in connection with the conveying system (IF, EW, SW1, IW, TW1, MIC, TW2, CV1, SW2, CV2) so as to facilitate the conveying system (IF, EW, SW1, IW, TW1, MIC, TW2, CV1, SW2, CV2) to also transport the standard objects (SO; HVSO; LDSO; FCSO; ALSO; UFSO) to the inspection stations (IS 1 - ISn).

7. The inspection machine (ASIM) according to any of the claim 1 to 6, wherein the system test arrangement (SSO, SO, SW3, ECU) comprises at least two standard objects (SO; HVSO; LDSO; FCSO; ALSO; UFSO); wherein the control unit (ECU) is arranged to control an exposure of at least one of said at least two standard objects (SO; HVSO; LDSO; FCSO; ALSO; UFSO) to at least one of said one or more inspection stations (IS1 - ISn), and wherein said at least two standard objects (SO; HVSO; LDSO; FCSO; ALSO; UFSO) are arranged in a predetermined sequence to facilitate the control unit (ECU) to control or at least track the movement of the individual standard objects (SO; HVSO; LDSO; FCSO; ALSO; UFSO).

8. The inspection machine (ASIM) according to any of the claims 1 to 7, wherein the at least one standard object (SO; HVSO; LDSO; FCSO; ALSO; UFSO) comprises a machine readable identification to facilitate the one or more inspection stations (IS 1 - ISn) or the control unit (ECU) to identify the standard objects (SO; HVSO; LDSO; FCSO; ALSO; UFSO).

9. Method for testing the performance level of an inspection machine (ASIM) comprising one or more inspection stations (IS1 - ISn) comprising sub-components from the list of mechanical, optical and electrical sub-components, the inspection stations (IS1 - ISn) being arranged for inspecting containers (CT), such as vials, according to container analysis routines; the method comprising the steps of:
providing one or more standard objects (SO; HVSO; LDSO; FCSO; ALSO; UFSO) having one or more specific characteristics that are arranged to test specific of said sub-components of the one or more inspection stations (IS1 - ISn);
inspecting the standard objects (SO; HVSO; LDSO; FCSO; ALSO; UFSO) according to dedicated standard object analysis routines by at least one of the one or more inspection stations (IS1 - ISn) to establish standard object-dependent inspection results; a standard object analysis routine being a dedicated individual test algorithm different from a container analysis routine in at least one of the one or more inspection stations (IS1 - ISn);
receiving standard object-dependent inspection results from the at least one of the one or more inspection stations (IS1 - ISn); and
determining an inspection machine status on the basis of the standard object-dependent inspection results; wherein the inspection machine status represents information about said sub-components of said inspection stations (IS1 - ISn).

10. The method for testing according to claim 9, wherein the method comprises comparing the standard object-dependent inspection results with predetermined reference inspection results and basing the inspection machine status on the basis of a result of the comparison; wherein the reference inspection results are predetermined for different combinations of standard objects (SO; HVSO; LDSO; FCSO; ALSO; UFSO), inspection stations (IS1 - ISn) and standard object analysis routines.

11. The method for testing according to claim 9 or 10, wherein the inspection machine status represents one or more of fault margin information for inspection stations (IS1 - ISn) or sub-components thereof, mechanical parameters of said inspection machine (ASIM), optical parameters of said inspection machine (ASIM) and electrical or electronic data parameters of said inspection machine (ASIM).

12. The method for testing according to any of the claims 9 to 11, wherein the method comprises providing information for establishing correction guidance on the basis of said inspection machine status or for correcting an error or inaccuracy on the basis of said inspection machine status.

13. The method for testing according to any of the claims 9 to 12, wherein the step of providing the standard objects (SO; HVSO; LDSO; FCSO; ALSO; UFSO) for inspection involves conveying the standard objects (SO; HVSO; LDSO; FCSO; ALSO; UFSO) from a standard object storage (SSO) to the inspection stations (IS1 - ISn), and wherein the standard objects (SO; HVSO; LDSO; FCSO; ALSO; UFSO) are conveyed back to the standard object storage (SSO) after one or more standard object-dependent inspection results have been obtained by the inspection stations (IS 1 - Isn).

14. A set of standard objects for use in a test of an inspection machine (ASIM) comprising one or more inspection stations (IS1 - ISn) comprising sub-components from the list of mechanical, optical and electrical sub-components, the inspection stations (IS1 - ISn) being arranged for inspecting containers (CT) such as vials; the set of standard objects comprising one or more standard objects (SO; HVSO; LDSO; FCSO; ALSO; UFSO) and for each standard object (SO; HVSO; LDSO; FCSO; ALSO; UFSO) an associated reference inspection result for one or more of the inspection stations (IS1 - ISn) of the inspection machine (ASIM), wherein the standard objects (SO; HVSO; LDSO; FCSO; ALSO; UFSO) have one or more specific characteristics that are arranged to test specific of said sub-components of the one or more inspection stations (IS1 - ISn), wherein the standard objects (SO; HVSO; LDSO; FCSO; ALSO; UFSO) comprise machine readable identification to facilitate the one or more inspection stations (IS1 - ISn) or a control unit (ECU) of the inspection machine (ASIM) to identify the standard objects (SO; HVSO; LDSO; FCSO; ALSO; UFSO), wherein the machine readable identification includes standard object analysis routines or identifies a source for that information, and wherein the standard object analysis routines are dedicated individual test algorithms for inspecting the standard objects (SO; HVSO; LDSO; FCSO; ALSO; UFSO).

15. A system test arrangement (SSO, SO, SW3, ECU) for use in connection with an inspection machine (ASIM) comprising one or more inspection stations (IS1 - ISn) comprising sub-components from the list of mechanical, optical and electrical sub-components, the inspection stations (IS1 - ISn) being arranged for inspecting containers (CT) such as vials, the system test arrangement comprising one or more standard objects (SO; HVSO; LDSO; FCSO; ALSO; UFSO) according to claim 14.

## Patentansprüche

1. Inspektionsmaschine (ASIM) zum Inspizieren von Behältern (CT) wie Fläschchen, die Inspektionsmaschine (ASIM) umfassend:
eine oder mehrere Inspektionsstationen (IS1 - ISn), die Unterkomponenten von der Liste von mechanischen, optischen und elektrischen Unterkomponenten umfassen, wobei jede Inspektionsstation (IS1 - ISn) angeordnet ist, um eine Behälteranalyseroutine zum Inspizieren eines Behälters (CT) durchzuführen und um ein behälterabhängiges Inspektionsergebnis herzustellen;
eine Steuereinheit (ECU), die angeordnet ist, um die behälterabhängigen Inspektionsergebnisse zu analysieren, die sich auf den Behälter (CT) beziehen und von der einen oder den mehreren Inspektionsstationen (IS1 - ISn) erhalten werden, und auf der Basis davon zu bestimmen, ob der Behälter (CT) eine oder mehrere Grundlagen für eine Abweisung erfüllt; und
einen Sortierer (SRT), der mindestens teilweise durch die Steuereinheit (ECU) gesteuert wird, um den Behälter (CT) umzuleiten, falls die Steuereinheit (ECU) bestimmt hat, dass der Behälter (CT) eine oder mehrere Grundlagen für die Abweisung erfüllt;
**dadurch gekennzeichnet, dass** die Inspektionsmaschine (ASIM) ferner eine Systemtestanordnung (SSO, SO, SW3, ECU) in Verbindung mit der einen oder den mehreren Inspektionsstationen (IS1 - Isn) umfasst, die Systemtestanordnung (SSO, SO, SW3, ECU) umfassend mindestens ein Standardobjekt (SO; HVSO; LDSO; FCSO; ALSO; UFSO), das ein oder mehrere spezifische Merkmale aufweist, die angeordnet sind, um spezifische der Unterkomponenten der einen oder der mehreren Inspektionsstationen (IS1 - ISn) zu testen;
wobei mindestens eine der einen oder der mehreren Inspektionsstationen (IS1 - ISn) ferner angeordnet ist, um eine Standardobjektanalyseroutine durchzuführen, die sich von der Behälteranalyseroutine unterscheidet, und die ein dedizierter individueller Testalgorithmus zum Inspizieren eines Standardobjekts (SO; HVSO; LDSO; FCSO; ALSO; UFSO) ist, und um objektabhängige Standardinspektionsergebnisse herzustellen; wobei die Standardobjekte (SO; HVSO; LDSO; FCSO; ALSO; UFSO) und die Standardobjektanalyseroutinen angeordnet sind, um objektabhängige Standardinspektionsergebnisse herzustellen, die Statusinformationen über die Unterkomponenten der Inspektionsstationen (IS1 - ISn) darstellen; und
wobei die Inspektionsstation (IS1 - ISn) oder die Steuereinheit (ECU) angeordnet ist, um die objektabhängigen Standardinspektionsergebnisse zu analysieren, die sich auf das Standardobjekt (SO; HVSO; LDSO; FCSO; ALSO; UFSO) beziehen und von der mindestens einen der einen oder der mehreren Inspektionsstationen (IS1 - ISn) erhalten werden.

2. Inspektionsmaschine (ASIM) nach Anspruch 1, wobei die Inspektionsstation (IS1 - ISn) oder die Steuereinheit (ECU) angeordnet ist, um die objektabhängigen Standardinspektionsergebnisse mit Referenzinspektionsergebnissen zu vergleichen; wobei die Referenzinspektionsergebnisse für unterschiedliche Kombinationen von Standardobjekten (SO; HVSO; LDSO; FCSO; ALSO; UFSO), Inspektionsstationen (IS1 - ISn) und Standardobjektanalyseroutinen vorbestimmt sind.

3. Inspektionsmaschine (ASIM) nach Anspruch 2, wobei eines oder mehrere der Referenzinspektionsergebnisse multidimensional, z. B. ein Bild, oder multivariabel sind, z. B. umfassend mehrere Parameter.

4. Inspektionsmaschine (ASIM) nach einem der Ansprüche 1 bis 3, wobei die Standardobjekte (SO; HVSO; LDSO; FCSO; ALSO; UFSO) und die Standardobjektanalyseroutinen angeordnet sind, um objektabhängige Standardinspektionsergebnisse, die Fehlerspielrauminformationen darstellen, oder mindestens ein objektabhängiges Standardinspektionsergebnis herzustellen, das einen oder mehrere von mechanischen Parametern, optischen Parametern, elektrischen oder elektronischen Datenparametern und Zeitparametern der Inspektionsmaschine (ASIM) darstellt.

5. Inspektionsmaschine (ASIM) nach einem der Ansprüche 1 bis 4, wobei mindestens eines der Standardobjekte (SO; HVSO; LDSO; FCSO; ALSO; UFSO) mit einer oder mehreren spezifischen Merkmalen angeordnet ist, die einem oder mehreren spezifischen Testparametern entsprechen, die spezifisch durch eine oder mehrere der Inspektionsstationen (IS1 - ISn) getestet werden, und wobei eines oder mehrere der Standardobjekte (SO; HVSO; LDSO; FCSO; ALSO; UFSO) aus einer Liste ausgewählt werden, umfassend:
- Lichtverteilungsstandardobjekte (LDSO),
- Fokusstandardobjekte (FCSO, ALSO),
- Ausrichtungsstandardobjekte (ALSO),
- Entfaltungsstandardobjekte (UFSO),
- Hochspannungsstandardobjekte (HVSO),
- Vergrößerungsstandardobjekte,
- Schleuderdrehzahlstandardobjekte und
- Zeitstandardobjekte.

6. Inspektionsmaschine (ASIM) nach einem der Ansprüche 1 bis 5, wobei die Inspektionsmaschine (ASIM) ein Fördersystem (IF, EW, SW1, IW, TW1, MIC, TW2, CV1, SW2, CV2) zum Transportieren der Behälter (CT) zu den Inspektionsstationen (IS1 - ISn) und dem Sortierer (SRT) umfasst; und wobei die Systemtestanordnung (SSO, SO, SW3, ECU) einen Standardobjektspeicher (SSO) zum Halten der Standardobjekte (SO; HVSO; LDSO; FCSO; ALSO; UFSO) umfasst und in Verbindung mit dem Fördersystem (IF, EW, SW1, IW, TW1, MIC, TW2, CV1, SW2, CV2) angeordnet ist, um dem Fördersystem (IF, EW, SW1, IW, TW1, MIC, TW2, CV1, SW2, CV2) zu ermöglichen, um ebenso die Standardobjekte (SO; HVSO; LDSO; FCSO; ALSO; UFSO) zu den Inspektionsstationen (IS1 - ISn) zu transportieren.

7. Inspektionsmaschine (ASIM) nach einem der Ansprüche 1 bis 6, wobei die Systemtestanordnung (SSO, SO, SW3, ECU) mindestens zwei Standardobjekte (SO; HVSO; LDSO; FCSO; ALSO; UFSO) umfasst; wobei die Steuereinheit (ECU) angeordnet ist, um eine Belichtung von mindestens einem der mindestens zwei Standardobjekte (SO; HVSO; LDSO; FCSO; ALSO; UFSO) zu mindestens einer der einen oder mehreren Inspektionsstationen (IS1 - Isn) zu steuern, und wobei die mindestens zwei Standardobjekte (SO; HVSO; LDSO; FCSO; ALSO; UFSO) in einer vorbestimmten Sequenz angeordnet sind, um der Steuereinheit (ECU) zu ermöglichen, die Bewegung der einzelnen Standardobjekte (SO; HVSO; LDSO; FCSO; ALSO; UFSO) zu verfolgen.

8. Inspektionsmaschine (ASIM) nach einem der Ansprüche 1 bis 7, wobei das mindestens eine Standardobjekt (SO; HVSO; LDSO; FCSO; ALSO; UFSO) eine maschinenlesbare Identifikation umfasst, um der einen oder den mehreren Inspektionsstationen (IS1 - ISn) oder der Steuereinheit (ECU) zu ermöglichen, um die Standardobjekte (SO; HVSO; LDSO; FCSO; ALSO; UFSO) zu identifizieren.

9. Verfahren zum Testen des Leistungsniveaus einer Inspektionsmaschine (ASIM), umfassend eine oder mehrere Inspektionsstationen (IS1 - ISn), umfassend Unterkomponenten von der Liste von mechanischen, optischen und elektrischen Unterkomponenten, wobei die Inspektionsstationen (IS1 - ISn) zum Inspizieren von Behältern (CT), wie Fläschchen, gemäß Behälteranalyseroutinen angeordnet sind; das Verfahren umfassend die Schritte:
Bereitstellen eines oder mehrerer Standardobjekte (SO; HVSO; LDSO; FCSO; ALSO; UFSO), die ein oder mehrere spezifische Merkmale aufweisen, die angeordnet sind, um spezifische der Unterkomponenten der einen oder der mehreren Inspektionsstationen (IS1 - ISn) zu testen;
Inspizieren der Standardobjekte (SO; HVSO; LDSO; FCSO; ALSO; UFSO) gemäß dedizierten Standardobjektanalyseroutinen durch mindestens eine der einen oder der mehreren Inspektionsstationen (IS1 - ISn), um objektabhängige Standardinspektionsergebnisse herzustellen; wobei eine Standardobjektanalyseroutine ein dedizierter Einzeltestalgorithmus ist, der sich von einer Behälteranalyseroutine in mindestens einer der einen oder der mehreren Inspektionsstationen (IS1 - ISn) unterscheidet;
Empfangen von objektabhängigen Standardinspektionsergebnissen von der einen oder den mehreren Inspektionsstationen (IS1 - ISn); und
Bestimmen eines Inspektionsmaschinenstatus auf der Basis der objektabhängigen Standardinspektionsergebnisse; wobei der Inspektionsmaschinenstatus Informationen über die Unterkomponenten der Inspektionsstationen (IS1 - ISn) darstellt.

10. Verfahren zum Testen nach Anspruch 9, wobei das Verfahren das Vergleichen der objektabhängigen Standardinspektionsergebnisse mit vorbestimmten Referenzinspektionsergebnissen und das Basieren des Inspektionsmaschinenstatus auf der Basis eines Ergebnisses des Vergleichs umfasst; wobei die Referenzinspektionsergebnisse für unterschiedliche Kombinationen von Standardobjekten (SO; HVSO; LDSO; FCSO; ALSO; UFSO), Inspektionsstationen (IS1 - ISn) und Standardobjektanalyseroutinen vorbestimmt sind.

11. Verfahren zum Testen nach Anspruch 9 oder 10, wobei der Inspektionsmaschinenstatus eine oder mehrere Fehlerspielrauminformationen für Inspektionsstationen (IS1 - ISn) oder Unterkomponenten davon, mechanische Parameter der Inspektionsmaschine (ASIM), optische Parameter der Inspektionsmaschine (ASIM) und elektrische oder elektronische Datenparameter der Inspektionsmaschine (ASIM) darstellt.

12. Verfahren zum Testen nach einem der Ansprüche 9 bis 11, wobei das Verfahren das Bereitstellen von Informationen zum Herstellen einer Korrekturführung auf der Basis des Inspektionsmaschinenstatus oder zum Korrigieren eines Fehlers oder einer Ungenauigkeit auf der Basis des Inspektionsmaschinenstatus umfasst.

13. Verfahren zum Testen nach einem der Ansprüche 9 bis 12, wobei der Schritt des Bereitstellens der Standardobjekte (SO; HVSO; LDSO; FCSO; ALSO; UFSO) für eine Inspektion das Fördern der Standardobjekte (SO; HVSO; LDSO; FCSO; ALSO; UFSO) von einem Standardobjektspeicher (SSO) zu den Inspektionsstationen (IS1 - Isn) umfasst und wobei die Standardobjekte (SO; HVSO; LDSO; FCSO; ALSO; UFSO) nach einem oder mehreren objektabhängigen Standardinspektionsergebnisse, die durch die Inspektionsstationen (IS1 - Isn) erhalten werden, an den Standardobjektspeicher (SSO) zurückgefördert werden.

14. Satz von Standardobjekten zur Verwendung bei einem Test einer Inspektionsmaschine (ASIM), umfassend eine oder mehrere Inspektionsstationen (IS1 - ISn), umfassend Unterkomponenten von der Liste von mechanischen, optischen und elektrischen Unterkomponenten, wobei die Inspektionsstationen (IS1 - ISn) zum Inspizieren von Behältern (CT), wie Fläschchen, angeordnet werden; der Satz von Standardobjekten umfassend ein oder mehrere Standardobjekte (SO; HVSO; LDSO; FCSO; ALSO; UFSO) und für jedes Standardobjekt (SO; HVSO; LDSO; FCSO; ALSO; UFSO) ein zugehöriges Referenzinspektionsergebnis für eine oder mehrere der Inspektionsstationen (IS1 - ISn) der Inspektionsmaschine (ASIM), wobei die Standardobjekte (SO; HVSO; LDSO; FCSO; ALSO; UFSO) ein oder mehrere spezifische Merkmale aufweisen, die angeordnet sind, um spezifische der Unterkomponenten der einen oder der mehreren Inspektionsstationen (IS1 - ISn) zu testen, wobei die Standardobjekte (SO; HVSO; LDSO; FCSO; ALSO; UFSO) maschinenlesbare Identifikation umfassen, um der einen oder den mehreren Inspektionsstationen (IS1 - ISn) oder einer Steuereinheit (ECU) der Inspektionsmaschine (ASIM) zu ermöglichen, um die Standardobjekte (SO; HVSO; LDSO; FCSO; ALSO; UFSO) zu identifizieren, wobei die maschinenlesbare Identifikation Standardobjektanalyseroutinen einschließt oder eine Quelle für diese Informationen identifiziert, und wobei die Standardobjektanalyseroutinen dedizierte individuelle Testalgorithmen zum Inspizieren der Standardobjekte (SO; HVSO; LDSO; FCSO; ALSO; UFSO) sind.

15. Systemtestanordnung (SSO, SO, SW3, ECU) zur Verwendung in Verbindung mit einer Inspektionsmaschine (ASIM), umfassend eine oder mehrere Inspektionsstationen (IS1 - ISn), umfassend Unterkomponenten von der Liste von mechanischen, optischen und elektrischen Unterkomponenten, wobei die Inspektionsstationen (IS1 - ISn) zum Inspizieren von Behältern (CT), wie Fläschchen, angeordnet sind, die Systemtestanordnung umfassend ein oder mehrere Standardobjekte (SO; HVSO; LDSO; FCSO; ALSO; UFSO) nach Anspruch 14.

## Revendications

1. Machine d'inspection (ASIM) pour inspecter des récipients (CT) tels que des flacons, ladite machine d'inspection (ASIM) comprenant :
une ou plusieurs stations d'inspection (IS1 à ISn) comprenant des sous-composants de la liste de sous-composants mécaniques, optiques et électriques, chaque station d'inspection (IS1 à ISn) étant agencée pour mettre en oeuvre une routine d'analyse de récipient pour inspecter un récipient (CT) et pour établir un résultat d'inspection dépendant du récipient ;
une unité de commande (ECU) agencée pour analyser lesdits résultats d'inspection dépendant du récipient se rapportant au récipient (CT) et obtenus à partir des une ou plusieurs stations d'inspection (IS1 à ISn), et en fonction de ceux-ci, déterminer si le récipient (CT) remplit un ou plusieurs motifs de rejet ; et
une trieuse (SRT) au moins partiellement commandée par l'unité de commande (ECU) pour dévier le récipient (CT) si l'unité de commande (ECU) a déterminé que le récipient (CT) remplit un ou plusieurs motifs de rejet ;
**caractérisée en ce que** la machine d'inspection (ASIM) comprend en outre un agencement de test de système (SSO, SO, SW3, ECU) en liaison avec les une ou plusieurs stations d'inspection (IS1 à ISn), l'agencement de test de système (SSO, SO, SW3, ECU) comprenant au moins un objet étalon (SO ; HVSO ; LDSO ; FCSO ; ALSO ; UFSO) ayant une ou plusieurs caractéristiques spécifiques qui sont agencées pour tester des spécifiques desdits sous-composants des un ou plusieurs stations d'inspection (IS1 à ISn) ;
dans laquelle au moins une des une ou plusieurs stations d'inspection (IS1 à ISn) est en outre agencée pour effectuer une routine d'analyse d'objet étalon, qui est différente de la routine d'analyse de récipient, et qui est un algorithme de test individuel dédié pour inspecter un objet étalon (SO ; HVSO ; LDSO ; FCSO ; ALSO ; UFSO) et pour établir un résultat d'inspection dépendant de l'objet étalon ; lesdits objets étalons (SO ; HVSO ; LDSO ; FCSO ; ALSO ; UFSO) et lesdites routines d'analyse d'objet étalon étant agencés pour établir des résultats d'inspection dépendant de l'objet étalon qui représentent des informations de statut concernant lesdits sous-composants desdites stations d'inspection (IS1 à ISn) ; et
dans laquelle ladite station d'inspection (IS1 à ISn) ou unité de commande (ECU) est agencée pour analyser lesdits résultats d'inspection dépendant de l'objet étalon se rapportant à l'objet
étalon (SO ; HVSO ; LDSO ; FCSO ; ALSO ; UFSO) et obtenus à partir de l'au moins une des une ou plusieurs stations d'inspection (IS1 à ISn).

2. Machine d'inspection (ASIM) selon la revendication 1, dans laquelle ladite station d'inspection (IS1 à ISn) ou unité de commande (ECU) est agencée pour comparer lesdites résultats d'inspection dépendant de l'objet étalon à des résultats d'inspection de référence ; les résultats d'inspection de référence étant prédéterminés pour différentes combinaisons d'objets étalons (SO ; HVSO ; LDSO ; FCSO ; ALSO ; UFSO), de stations d'inspection (IS1 à ISn) et de routines d'analyse d'objet étalon.

3. Machine d'inspection (ASIM) selon la revendication 2, dans laquelle un ou plusieurs desdits résultats d'inspection de référence sont multidimensionnels, e.g. une image, ou multi-variables, e.g. comprenant plusieurs paramètres.

4. Machine d'inspection (ASIM) selon l'une quelconque des revendications 1 à 3, dans laquelle lesdits objets étalons (SO ; HVSO ; LDSO ; FCSO ; ALSO ; UFSO) et lesdites routines d'analyse d'objet étalon sont agencées pour établir des résultats d'inspection dépendant de l'objet étalon qui représentent des informations de marge de défaut, ou au moins un résultat d'inspection dépendant de l'objet étalon qui représente un ou plusieurs de paramètres mécaniques, paramètres optiques, paramètres de données électriques ou électroniques et paramètres de synchronisation de ladite machine d'inspection (ASIM).

5. Machine d'inspection (ASIM) selon l'une quelconque des revendications 1 à 4, dans laquelle au moins l'un des objets étalons (SO ; HVSO ; LDSO ; FCSO ; ALSO ; UFSO) est agencé avec une ou plusieurs caractéristiques spécifiques qui correspondent à un ou plusieurs paramètres de test spécifiques qui sont spécifiquement testés par une ou plusieurs des stations d'inspection (IS1 à ISn), et dans laquelle un ou plusieurs des objets étalons (SO ; HVSO ; LDSO ; FCSO ; ALSO ; UFSO) est sélectionné dans une liste comprenant :
- des objets étalons de distribution de lumière (LDSO),
- des objets étalons de mise au point (FCSO, ALSO),
- des objets étalons d'alignement (ALSO),
- des objets étalons de déploiement (UFSO),
- des objets étalons à haute tension (HVSO),
- des objets étalons d'agrandissement,
- des objets étalons de vitesse de spin, et
- des objets étalons de temporisation.

6. Machine d'inspection (ASIM) selon l'une quelconque des
revendications 1 à 5, dans laquelle la machine d'inspection (ASIM) comprend un système de convoyage (IF, EW, SW1, IW, TW1, MIC, TW2, CV1, SW2, CV2) pour transporter les récipients (CT) vers les stations d'inspection (IS1 - ISn) et la trieuse (SRT) ; et dans laquelle ledit agencement de test de
système (SSO, SO, SW3, ECU) comprend un stockage d'objet étalon (SSO) pour contenir les objets étalons (SO ; HVSO ; LDSO ; FCSO ; ALSO ; UFSO) et disposé en liaison avec le système de
convoyage (IF, EW, SW1, IW, TW1, MIC, TW2, CV1, SW2, CV2) de manière à faciliter au système de
convoyage (IF, EW, SW1, IW, TW1, MIC, TW2, CV1, SW2, CV2) le transport également des objets étalons (SO ; HVSO ; LDSO ; FCSO ; ALSO ; UFSO) vers les stations d'inspection (IS1 à ISn).

7. Machine d'inspection (ASIM) selon l'une quelconque des revendications 1 à 6, dans laquelle l'agencement de test de
système (SSO, SO, SW3, ECU) comprend au moins deux objets étalons (SO ; HVSO ; LDSO ; FCSO ; ALSO ; UFSO) ; dans laquelle l'unité de commande (ECU) est agencée pour commander une exposition d'au moins l'un desdits au moins deux objets étalons (SO ; HVSO ; LDSO ; FCSO ; ALSO ; UFSO) vers au moins l'une desdites une ou plusieurs stations d'inspection (IS1 à ISn), et dans laquelle lesdits au moins deux objets étalons (SO ; HVSO ; LDSO ; FCSO ; ALSO ; UFSO) sont agencés dans une séquence prédéterminée pour faciliter à l'unité de commande (ECU) la commande ou au moins le suivi du mouvement des objets étalons (SO ; HVSO ; LDSO ; FCSO ; ALSO ; UFSO) individuels.

8. Machine d'inspection (ASIM) selon l'une quelconque des revendications 1 à 7, dans laquelle l'au moins un objet étalon (SO ; HVSO ; LDSO ; FCSO ; ALSO ; UFSO) comprend une identification lisible par machine pour faciliter aux une ou plusieurs stations d'inspection (IS1 à ISn) ou à l'unité de commande (ECU) l'identification des objets étalons (SO ; HVSO ; LDSO ; FCSO ; ALSO ; UFSO).

9. Procédé de test du niveau de performance d'une machine d'inspection (ASIM) comprenant une ou plusieurs stations d'inspection (IS1 à ISn) comprenant des sous-composants de la liste de sous-composants mécaniques, optiques et électriques, les stations d'inspection (IS1 à ISn) étant agencées pour inspecter des récipients (CT), tels que des flacons, selon des routines d'analyse de récipients ; le procédé comprenant les étapes consistant à :
fournir un ou plusieurs objets étalons (SO ; HVSO ; LDSO ; FCSO ; ALSO ; UFSO) ayant une ou plusieurs caractéristiques spécifiques qui sont agencées pour tester des spécifiques desdits sous-composants des un ou plusieurs stations d'inspection (IS1 à ISn) ;
inspecter les objets étalons (SO ; HVSO ; LDSO ; FCSO ; ALSO ; UFSO) selon des routines d'analyse d'objet étalon dédiées par au moins l'une des une ou plusieurs stations d'inspection (IS1 à ISn) pour établir des résultats d'inspection dépendant de l'objet étalon ; une routine d'analyse d'objet étalon étant un algorithme de test individuel dédié différent d'une routine d'analyse de récipient dans au moins l'une des une ou plusieurs stations d'inspection (IS1 à ISn) ;
recevoir des résultats d'inspection dépendant de l'objet étalon provenant de l'au moins une des une ou plusieurs stations d'inspection (IS1 à ISn) ; et
déterminer un statut de machine d'inspection en fonction des résultats d'inspection dépendant de l'objet étalon ; dans lequel le statut de machine d'inspection représente des informations concernant lesdits sous-composants desdites stations d'inspection (IS1 à ISn).

10. Procédé de test selon la revendication 9, dans lequel le procédé comprend la comparaison des résultats d'inspection dépendant de l'objet étalon avec des résultats d'inspection de référence prédéterminés et le fait de baser le statut de machine d'inspection en fonction d'un résultat de la comparaison ; dans lequel les résultats d'inspection de référence sont prédéterminés pour différentes combinaisons d'objets étalons (SO ; HVSO ; LDSO ; FCSO ; ALSO ; UFSO), de stations d'inspection (IS1 à ISn) et de routines d'analyse d'objet étalon.

11. Procédé de test selon la revendication 9 ou 10, dans lequel le statut de machine d'inspection représente une ou plusieurs des informations de marge de défaut pour des stations d'inspection (IS1 à ISn) ou des sous-composants de celles-ci, des paramètres mécaniques de ladite machine d'inspection (ASIM), des paramètres optiques de ladite machine d'inspection (ASIM) et des paramètres de données électriques ou électroniques de ladite machine d'inspection (ASIM).

12. Procédé de test selon l'une quelconque des revendications 9 à 11, dans lequel le procédé comprend la fourniture d'informations pour établir un guidage de correction en fonction dudit statut de machine d'inspection ou pour corriger une erreur ou imprécision en fonction dudit statut de machine d'inspection.

13. Procédé de test selon l'une quelconque des revendications 9 à 12, dans lequel l'étape de fourniture des objets étalons (SO ; HVSO ; LDSO ; FCSO ; ALSO ; UFSO) pour inspection consiste à convoyer les objets étalons (SO ; HVSO ; LDSO ; FCSO ; ALSO ; UFSO) à partir d'un stockage d'objet étalon (SSO) vers les stations d'inspection (IS1 à ISn), et dans lequel les objets étalons (SO ; HVSO ; LDSO ; FCSO ; ALSO ; UFSO) sont convoyés en retour vers le stockage d'objet étalon (SSO) après qu'un ou plusieurs résultats d'inspection dépendant de l'objet étalon ont été obtenus par les stations d'inspection (IS1 à Isn).

14. Ensemble d'objets étalons pour utilisation dans un test d'une machine d'inspection (ASIM) comprenant une ou plusieurs stations d'inspection (IS1 à ISn) comprenant des sous-composants de la liste de sous-composants mécaniques, optiques et électriques, les stations d'inspection (IS1 à ISn) étant agencées pour inspecter des récipients (CT) tels que des flacons ; l'ensemble d'objets étalons comprenant un ou plusieurs objets étalons (SO ; HVSO ; LDSO ; FCSO ; ALSO ; UFSO) et pour chaque objet étalon (SO ; HVSO ; LDSO ; FCSO ; ALSO ; UFSO) un résultat d'inspection de référence associé pour une ou plusieurs des stations d'inspection (IS1 à ISn) de la machine d'inspection (ASIM), dans lequel les objets étalons (SO ; HVSO ; LDSO ; FCSO ; ALSO ; UFSO) ont une ou plusieurs caractéristiques spécifiques qui sont agencées pour tester des spécifiques desdits sous-composants des une ou plusieurs stations d'inspection (IS1 à ISn), dans lequel les objets étalons (SO ; HVSO ; LDSO ; FCSO ; ALSO ; UFSO) comprennent une identification lisible par machine pour faciliter aux une ou plusieurs stations d'inspection (IS1 à ISn) ou à une unité de commande (ECU) de la machine d'inspection (ASIM) l'identification des objets étalons (SO ; HVSO ; LDSO ; FCSO ; ALSO ; UFSO), dans lequel l'identification lisible par machine comporte des routines d'analyse d'objet étalon ou identifie une source pour cette information, et dans lequel les routines d'analyse d'objet étalon sont des algorithmes de test individuels dédiés pour inspecter les objets étalons (SO ; HVSO ; LDSO ; FCSO ; ALSO ; UFSO).

15. Agencement de test de système (SSO, SO, SW3, ECU) destiné à être utilisé en liaison avec une machine d'inspection (ASIM) comprenant une ou plusieurs stations d'inspection (IS1 à ISn) comprenant des sous-composants de la liste de sous-composants mécaniques, optiques et électriques, les stations d'inspection (IS1 à ISn) étant agencées pour inspecter des récipients (CT) tels que des flacons, l'agencement de test de système comprenant un ou plusieurs objets étalons (SO ; HVSO ; LDSO ; FCSO ; ALSO ; UFSO) selon la revendication 14.
